# EUROPEAN PATENT APPLICATION

(11) **EP 1 431 287 A1**
(43) Date of publication of application: **23.06.2004**
(21) Application number: 03380293.5
(22) Date of filing: 16.12.2003
(51) Int. Cl.: C07D 211/22, C12P 17/12, C12P 41/00

(54) **Optically pure paroxetine precursos**

(30) Priority: 18.12.2002 ES 200202916
(71) Applicant: Astur-Pharma, S.A., Llanera 33192, Asturias (ES)
(72) Inventor: The designation of the inventor has not yet been filed
(74) Representative: Isern Jara, Nuria

(57) **Abstract**

A biocatalytic process to obtain optically enriched precursors of trans-4-(4-fluorophenyl)-3-hydroxymethylpiperidines,based on the enzymatic resolution of racemic derivatives of general formula III (where R³ is preferably phenyl or benzyl) using cyclic anhydrydes or the Meldrum's acid (2,2-dimethyl-1,3-dioxane-4,6-dione) as acylating agents of the hydroxyl group. Depending on the enzyme and the chosen reaction conditions, either of the two enantiomers may be obtained with high enantiomeric purity. These compounds are important intermediates in the synthesis of the paroxetine anti-depressive agent.

## Description

### Field of the invention

Paroxetine is an arylpiperidine acting as a selective inhibitor for serotonin (5-HT) re-uptake. Mainly, this compound has anti-depressive and anti-parkinsonian activity. Only the *trans*-(-) isomer, with absolute configuration (3*S*,4*R*) may be used as a drug.

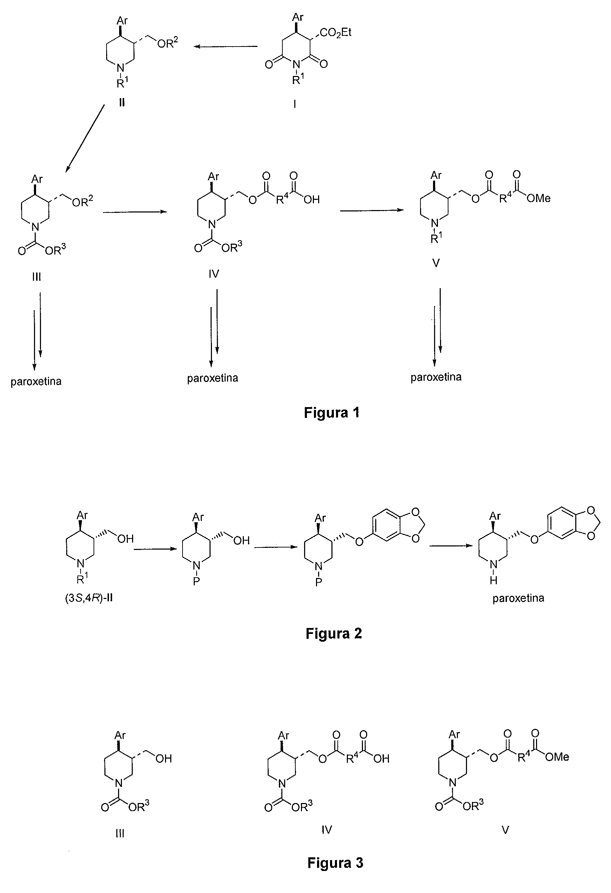

The object of the present invention is a biocatalytic process to obtain optically enriched derivatives of *trans*-4-(4-fluorophenyl)-3-hydroxymethylpiperidines with formulas II, III, IV and V, wherein Ar is a phenyl group, substituted by one or more halogen atoms, R¹ is hydrogen, alkyl or aralkyl, R² is hydrogen, R³ is alkyl, alkenyl, aralkyl or aryl, R⁴ is, alkyl, alkenyl, alkyloxyalkyl or aryl (Figure 1). These are important intermediates in the synthesis of paroxetine.

Another object of the invention are the new compounds obtained, IV and V both in the racemic and enatiomerically enriched forms, suitable for the synthesis of paroxetine, as well as their preparation processes.

### Prior State of the art

The piperidinecarbinol compounds of formula II, were described for the first time, together with their preparation, in US patents 3,912,743 and 4,007,196 (R¹=hydrogen or methyl and R²=hydrogen). Paroxetine is prepared from said optically pure precursors (3*S*, *4R*)-*trans*-II by means of the process described in US patent 4,007,196. In patent application EP 812 827-A1 and in *Tetrahedron: Asymm.* **1996**, 7, 1591, the preparation of paroxetine from a piperidinecarbinol of formula II is described, with R¹= hydrogen (Figure 2; P=R¹ or other protector group).

In US patents 4,007,196 and 4,902,801, said piperidinecarbinol II compound is firstly obtained as a racemic mixture and then resolved in its enantiomers by conventional methods, such as the crystallisation of its diastereoisomer salts with chiral acids, like (+)-tartaric, (+)-2'-nitrotartranylic or (-)-di-*p*-toluyltartaric. The drawback of this process is that crystallisation is cumbersome and relatively expensive.

An alternative to said crystallisation of diastereoisomer salts are the biocatalytic enantiomer separation methods.

In US patent 5,258,517 and patent applications WO 93/22284-A1 and WO 98/02556-A3, resolution methods are described for enantiomers of the intermediate I (R¹= Me) by means of enzymatic or microbial hydrolysis. After resolution, the imidoester (3*S*, 4*R*)-*trans*-1, enantiomerically enriched, is reduced by conventional methods to piperidinecarbinol (3*S*, 4*R*)-*trans*-II.

In patent application WO 98/53824-A1, the preparation of optically enriched (3*S*, 4*R*)-*trans*-II (R¹=benzyl) is described, by means of synthesis from an enantiopure precursor, in turn, obtained by means of the enzymatic asymmetrisation of dimethyl 3-(4-fluorophenyl)-glutarate.

In patent application ES 200101658 and in *J. Org. Chem* **2001**, *66*, 8947-8953, methods of resolution of the intermediate *trans*-III, by means of enzymatic acylation reactions using hydrolases are described. After resolution, both the piperidinecarbinol (3*S*, 4*R*)-*trans*-III and its acylated derivatives of (3*S*, 4*R*) configuration are used in the paroxetine synthesis.

On the other hand, derivatives of the piperidinecarbonil carbamate type (general formula III) are described in patent applicationsWO97/24323-A1, EP 812 827-A1 and in WO98/53824-A1. In all these cases, the optically enriched compounds (3*S*, 4*R*)-*trans*-III are obtained by means of conventional reactions from an optically pure (3*S*, 4*R*)-*trans*-II compound with R² equal to hydrogen. In said patents, paroxetine synthesis is described as from precursors of formula III with R² equal to hydrogen and different R³ substituents.

### General Description of the Invention

For the first time, the present invention describes new compounds of formulas *trans*-IV and *trans*-V, both in the racemic and enantiomerically enriched forms (Figure 3), where:
■ R¹: hydrogen, alkyl or aralkyl.
■ R³: alkyl, haloalkyl, alkenyl, aralkyl or aryl.
■ R⁴: alkyl, alkenyl,alkyloxyalkyl or aryl.
■ Ar: Phenyl group substituted with one or more halogen atoms.

Another object of the present invention is an enzymatic resolution process for piperidinecarbinol derivatives of formula (±)-*trans*-III, in turn permitting to obtain compounds with enantiomerically enriched structures III and IV. Said process consists of the enantioselective acylation -catalysed by an enzyme- of the hydroxymethyl group of the (±)-*trans*-III compound, using cyclic anhidrides or the Meldrum's acid (2,2-dimethyl-1,3-dioxan-4,6-dione), and stopping the reaction at a determinated conversion less than 100%, to give a compound of *trans*-IV formula. This process has two variants, permitting the attainment, as required, of either of the two enantiomers of the *trans*-III compound of enantiomierically enriched forms, whether
(3*S*, 4*R*)-III or (3*R*, 4*S*)-III. In the same process, the corresponding *trans*-IV isomer is also obtained with the opposite configuration (3*R*, 4*S*)-IV or (3*S*, 4*R*)-IV, respectively.

Another object of the present invention is also a preparation process of compounds with formula (3*S*, 4*R*)-III, as from compounds of formula (3*S*, 4*R*)-IV.

Another object of the present invention is also a process to prepare compounds with formula (3*S*, 4*R*)-III from compounds of formula (3*S*, 4*R*)-V.

Another object of the present invention is a process to prepare *trans*-V formula compounds from trans-IV formula compounds, both in the racemic form and in the enantiomerically enriched form.

The piperidinecarbinol derivatives of formula III with configuration (3*S*, 4*R*) may be used to obtain paroxetine by known methods, already described in the literature.

### Detailed Description of the Invention

The process object of the present invention, consists of making a racemic compound of formula *trans*-III react with a cyclic anhydryde in the presence of an enzyme, where R³ and R⁴ represent that mentioned previously. By action of the enzyme, one of the enantiomers of the substrate is selectively acylated, obtaining the *trans*-IV ester, while the other enantiomer mainly remains without acylation.

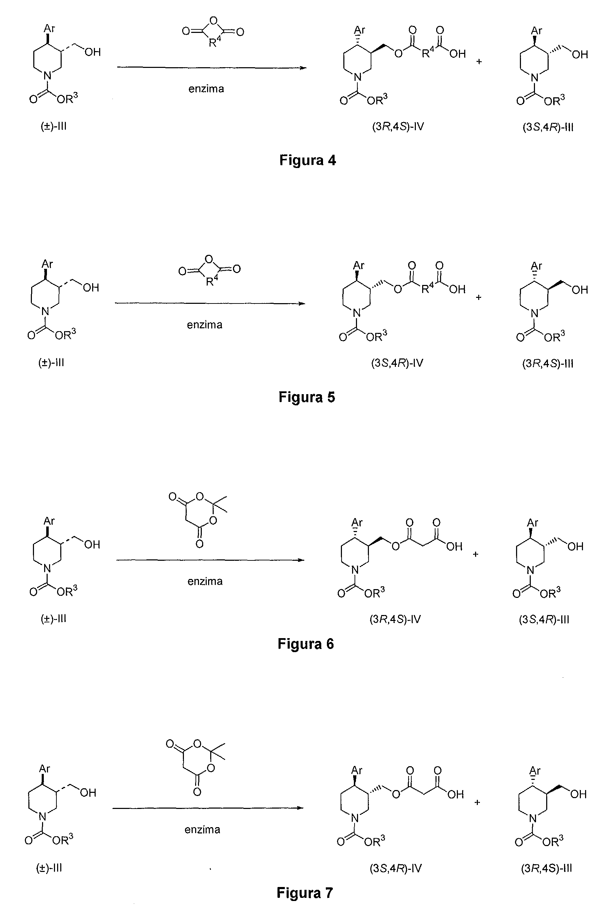

In the first variant of the process (Figure 4), the enzyme preferably catalyses the acylation of the enantiomer with configuration (3*R*, 4*S*)-III giving a compound (3*R*, 4*S*)-IV, while the isomer (3*S*, 4*R*)-III mainly remains without acylation.

In a second variant of the process (Figure 5), the enzyme preferably catalyses the acylation of the enantiomer with configuration (3*S*, 4*R*)-III, giving a compound (3*S*, 4*R*)-IV, while the isomer (3*R*, 4*S*)-III, mainly remains without acylation.

In other variant of the present invention the racemic compound of formula *trans*-III is made react with the Meldrum's acid in the presence of an enzyme, where R³ represents that mentioned previously. By action of the enzyme, one of the enantiomers of the substrate is selectively acylated, obtaining the *trans*-IV ester, while the other enantiomer mainly remains without acylation.

In the first variant of the process (Figure 6), the enzyme preferably catalyses the acylation of the enantiomer with configuration (3*R*, 4*S*)-III giving a compound (3*R*, 4*S*)-IV, while the isomer (3*S*, 4*R*)-III mainly remains without acylation.

In a second variant of the process (Figure 7), the enzyme preferably catalyses acylation of the enantiomer with configuration (3*S*, 4*R*)-III, giving a compound (3*S*, 4*R*)-IV, while the isomer (3*R*, 4*S*)-III, mainly remains without acylation.

In either of the two variants of the enzymatic process, when the desired conversion is reached, normally about 50%, the reaction is stopped -for example, by filtering the enzyme- and the resulting compounds are separated by means of an extraction in basic medium. It should be considered that the choice of the enzyme and the specific reaction conditions determine which of the enantiomers is preferably acylated.

When the reaction is sufficiently enantioselective, the reaction conversion should be about 50% to obtain the maximum yield of acylated product and remaining optically enriched substrate. However, when the enantioselectivity is moderate, other conversion values may be preferable to ensure an enantiomeric excess value, sufficiently high for any of the components of the reaction mixture. For example, it is known that as conversion increases under the given reaction conditions, the enantiomeric excess of the remaining substrate gradually increases and the enantiomeric excess of the product gradually decreases. The specific conversion value at which the reaction should be stopped will depend on the enantioselectivity of each specific case and the optical purity requirements of the products. Said value is determined by a known method by an expert in the matter, as described, for example in J. Amer. Chem. Soc. 1982, 104, 7294.

The process is carried out by dissolving the substrate in a suitable solvent and adding the enzyme and acylating agent. As a reaction medium, a pure organic solvent or mixture of organic solvents may be used. Suitable solvents may be aliphatic or aromatic hydrocarbon, esters, open or cyclic ethers, alcohols, etc

For the acylating agent, cyclic anhydrydes or the Meldrum's acid are used. This include alkyl, alkenyl, alkyloxyalkyl and aryl cyclic anhydrydes. The mole ratio of anhydryde and substrate may be equal, less or greater than 1:1.

Enzymes suitable to catalyse this process are the hydrolases, both of animal and microbial origin, in pure or semipurified form, free or immobilised. A special object of the present invention is the use of lipases. Some of these lipases come from a micro-organism of the generes *Candida, Rhizomucor, Pseudomonas* or *Aspergillus.*

The reaction should be performed between -20 and 100°C, preferably temperatures being between 0°C and 60°C.

It is a known fact, described for example in *Tetrahedrom* **2000**, *56*, 2905, that certain additives like triethylamine or crown ethers, among others, may be beneficial for the course of the enzymatic reaction.

In the procedure of the present invention, the final enantiomeric excess of both obtained componds IV and III, is greater than 60%, and even more concretely, greater than 95.

According to the first variant of the process, once the (3*S*, 4*R*)-III isomer has been obtained with high enantiomieric richness, this may be transformed into paroxetine by following already known processes, like the mesylation of the hydroxymethyl group followed by the nucleophillic attack of the 3,4-(methylenedioxy)phenol and final deprotection of nitrogen.

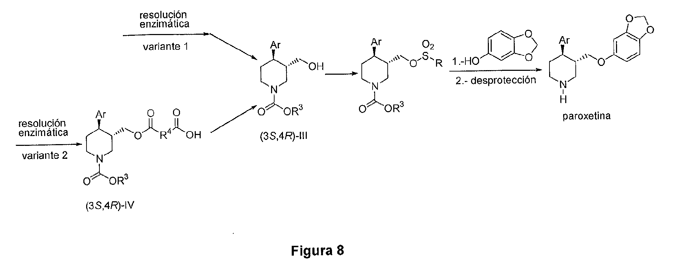

However, in the second variant of the enzymatic process, the enantiomer having the correct configuration to be converted into paroxetine, the (3*S*, 4*R*), is acylated by the enzyme to give optically enriched (3*S*, 4*R*)-IV. After separation of the compounds (3*S*, 4*R*)-IV and (3*R*, 4*S*)-III from the reaction mixture, in order to continue with the synthesis it is necessary to deacylate the hydroxymethyl, group of (3*S*, 4*R*)-IV, hence transforming it into (3*S*, 4*R*)-III. This compound (3*S*, 4*R*)-III with high enantiomeric richness, may be converted into paroxetine by following already known processes, like mesylation of the hydroxymethyl group followed by the nucleophillic attack of the 3,4-(methylenedioxy)phenol and final deprotection of the nitrogen. (See Figure 8, Ar = *p*-fluorophenyl).

In general, formula III compounds, both in the racemic and enantiomerically enriched forms, may also be obtained from those of structure IV and V by means of known oxygen deprotection methods (for example, acid or alkaline hydrolysis), choosing the most suitable method in each case according to R³ and R⁴ structures.

The formula V compounds, both in the racemic and enantiomerically enriched forms, may be obtained from those of structure IV by means of known esterification of carboxylic acid methods, choosing the most suitable method in each case according to R³ and R⁴ structures.

It may occur that in the optically enriched compounds of formula III, the most suitable nitrogen protector group for the enzymatic resolution is not convenient for continuation of the synthesis. In this case, after the enzymatic reaction, the first protector group may be eliminated -giving compounds of formula II- and introducing a new one, aspect which is included in the scope of this invention.

Another object of the present invention are the new acylated compounds in the 3-hydroxymethyl group of the piperidine ring, with formulas IV and V, both in the racemic and enantiomerically enriched forms.

Any new intermediate obtained following the process of this invention constitutes a further aspect of the invention. The processes and products of this invention may be applied to prepare active compounds described in the US patents 3,912,743 and US 4,007,196 and preferably to prepare paroxetine.

For a better understanding of the process object of the present invention, the following examples are given, which should be taken as not limiting the scope of the invention.

### Examples

### Example 1

### (±)-trans-3-[(3-Carboxypropanoyl)oxymethyl]-4-(4'-fluorophenyl)-N-phenyloxycarbonylpiperidine. (IV, R³ = Ph, R⁴ = (CH₂)₂)

1 g of (±)-*trans*-4-(4'-Fluorophenyl)-3-hydroxymethyl-*N*-phenyloxycarbonylpiperidine is dissolved in 40 ml dichloromethane and 1 mL pyridine. 1.21 g succinic anhydryde are added and the mixture is stirred at 50°C for 24 h. After that, Dowex 50 x 4-400 was added until slightly acid pH. The Dowex is removed by filtration and washed with dichloromethane. The organic phase is washed several times with a 10% aqueous solution of sodium chloride and concentrated to dryness to obtain the desired product. Yield: 1.04 g (80%). Hygroscopic solid.
- IR (cm⁻¹): 3500-2600, 1734, 1717, 1605, 1510.
- ¹H-NMR (CDCl₃), δ (ppm): 1.78-1.92 (m, 2H); 2.06-2.14 (m, 1H); 2.45-2.62 (m, 5H); 2.82-3.16 (m, 2H); 3.71 (dd, 1H); 3.90-3.97 (m, 1H); 4.34-4.56 (m, 2H); 6.24 (br s, 1H); 7.03 (dd, 2H); 7.13-7.25 (m, 5H); 7.40 (t, 2H).
- ¹³C-NMR (CDCl₃), δ (ppm): 28.7 (2C, CH₂); 34.1 (CH₂); 40.8 (CH); 44.3 (CH); 44.9 (CH₂); 47.4 (CH₂); 64.6 (CH₂); 115.6 (CH); 121.6 (CH); 125.3 (CH); 128.5 (CH); 129.2 (CH); 138.1 (C); 151.2 (C); 153.7 (CO); 161.6 (C); 171.6 (CO); 175.9 (CO).
- E.M.-E.S.I+: 430 (M + H, 7%); 452 (M + Na, 100); 468 (M + K, 48)

### Example 2

### (±)-trans-3-[(4-Carboxybutanoyl)oxymethyl]-4-(4'-fluorophenyl)-N-phenyloxycarbonylpiperidine. (IV, R³ = Ph, R⁴ = ((CH₂)₃)

1 g of (±)-*trans*-4-(4'-Fluorophenyl)-3-hydroxymethyl-*N*-phenyloxycarbonylpiperidine is dissolved in 40 ml dichloromethane and 1 mL pyridine. 1.39 g glutaric anhydryde are added and the mixture is stirred at 50°C for 24 h. After that, Dowex 50 x 4-400 was added until slightly acid pH. The Dowex is removed by filtration and washed with dichloromethane. The organic phase is washed several times with a 10% aqueous solution of sodium chloride and concentrated to dryness to obtain the desired product. Yield: 0.99 g (74%). Hygroscopic solid.
- IR (cm⁻¹): 3400-2600, 1738, 1712, 1604, 1512.
- ¹H-NMR (CDCl₃), δ (ppm): 1.76-1.95 (m, 4H); 2.11-2.22 (m, 1H); 2.34 (t, 4H);2.49-2.59 (m, 1H); 2.72-3.06 (m, 2H); 3.69 (dd, 1H); 3.82-3.92 (m, 1H); 4.43-4.56 (m, 2H); 7.03 (dd, 2H); 7.12-7.24 (m, 5H); 7.38 (t, 2H); 7.63 (br s, 1H).
- ¹³C-NMR (CDCl₃), δ (ppm): 19.8 (CH₂); 32.8 (2C, CH₂); 33.8 (CH₂); 40.6 (CH); 44.2 (CH); 44.6 (CH₂); 47.3 (CH₂); 64.2 (CH₂); 115.4 (CH); 121.5 (CH); 125.3 (CH); 128.5 (CH); 129.1 (CH); 138.1 (C); 151.0 (C); 153.6 (CO); 161.4 (C); 172.8 (CO); 176.4 (CO).
- E.M.-E.S.I+: 444 (M + H, 2%); 466 (M + Na, 100); 482 (M + K, 48).

### Example 3

### (±)-trans-3-[(4-Carboxy-3-oxabutanoyl)oxymethyl]-4-(4'-fluorophenyl)-N-phenyloxycarbonylpiperidine. (IV, R³ = Ph, R⁴ = CH₂OCH₂)

1 g of (±)-*trans*-4-(4'-Fluorophenyl)-3-hydroxymethyl-*N*-phenyloxycarbonylpiperidine is dissolved in 40 ml dichloromethane and 1 mL pyridine. 1.41 g diglycolic anhydryde are added and the mixture is stirred at 50°C for 24 h. After that, Dowex 50 x 4-400 was added until slightly acid pH. The Dowex is removed by filtration and washed with dichloromethane. The organic phase is washed several times with a 5% aqueous solution of sodium bicarbonate. The resulting aqueous phase is acidified with 1N chlorhydric acid and extracted with *tert*-butylmethylether. The organic phase is washed with a 10% aqueous solution of sodium chloride and concentrated to dryness to obtain the desired product. Yield: 0.92 g (68%). White solid, m.p: 144.5-146.3 °C.
- IR (cm⁻¹): 3445, 1699, 1510.
- ¹H-NMR (CDCl₃), δ (ppm): 1.74-1.92 (m,2H); 2.15-2.23 (m, 1H); 2.56 (td, 1H); 2.69-3.10 (m, 2H); 3.79 (dd, 1H); 3.95-4.23 (m, 5H); 4.32-4.53 (m, 2H); 7.01 (dd, 2H); 7.09-7.25 (m, 5H); 7.37 (t, 2H); 8.49 (br s, 1H).
- ¹³C-NMR (CDCl₃), δ (ppm): 34.2 (CH₂); 40.6 (CH); 44.4 (CH); 44.9 (CH₂); 47.3 (CH₂); 65.3 (CH₂); 68.2 (CH₂); 68.5 (CH₂); 115.7 (CH); 121.6 (CH); 125.4 (CH); 128.5 (CH); 129.2 (CH); 137.9 (C); 151.1 (C); 153.6 (CO); 161.7 (C); 169.8 (CO); 172.4 (CO).
- E.M.-E.S.I+: 446 (M + H, 7%); 468 (M + Na, 100); 484 (M + K, 100)

### Example 4

### (±)-trans-3-[(3-Carboxyprop-2-enoyl)oxymethyl]-4-(4'-fluorophenyl)-N-phenyloxycarbonylpiperidine. (IV, R³ = Ph, R⁴ = CH=CH)

1 g of (±)-*trans*-4-(4'-Fluorophenyl)-3-hydroxymethyl-*N*-phenyloxycarbonylpiperidine is dissolved in 40 ml dichloromethane and 1 mL pyridine. 1.19 g maleic anhydryde are added and the mixture is stirred at 50°C for 24 h. After that, Dowex 50 x 4-400 was added until slightly acid pH. The Dowex is removed by filtration and washed with dichloromethane. The organic phase is washed several times with a 5% aqueous solution of sodium bicarbonate. The resulting aqueous phase is acidified with 1N chlorhydric acid and extracted with *tert*-butylmethylether. The organic phase is washed with a 10% aqueous solution of sodium chloride and concentrated to dryness to obtain the desired product. Yield: 0.89 g (69%). Hygroscopic solid.
- IR (cm⁻¹): 3400-2500, 1748, 1714, 1648, 1604, 1510.
- ¹H-NMR (CDCl₃), δ (ppm): 1.78-1.96 (m,2H); 2.18-2.32 (m, 1H); 2.61 (td, 1H); 2.72-3.04 (m, 2H); 3.85 (dd, 1H); 4.03-4.09 (dd, 1H); 4.42-4.59 (m, 2H); 6.39 (br s, 1H); 6.73-6.93 (m, 2H); 7.04 (dd, 2H); 7.13-7.26 (m, 5H); 7.39 (t, 2H).
- ¹³C-NMR (CDCl₃), δ (ppm): 34.2 (CH₂); 40.9 (CH); 44.4 (CH); 44.9 (CH₂); 47.4 (CH₂); 65.4 (CH₂); 115.7 (CH); 121.6 (CH); 125.4 (CH); 128.5 (CH); 129.2 (CH); 133.2 (CH); 134.0 (CH); 138.0 (C); 151.2 (C); 153.8 (CO); 161.7 (C); 164.3 (CO); 168.4 (CO).
- E.M.-E.S.I+: 428 (M + H, 12%); 450 (M + Na, 100); 466 (M + K, 45)

### Example 5

### (±)-trans-3-[(2-Carboxybenzoyl)oxymethyl]-4-(4'-fluorophenyl)-N-phenyloxycarbonylpiperidine. (IV, R³ = Ph, R⁴ = o-phenylene)

1 g of (±)-*trans*-4-(4'-Fluorophenyl)-3-hydroxymethyl-*N*-phenyloxycarbonylpiperidine is dissolved in 40 ml dichloromethane and 1 mL pyridine. 1.80 g pftalic anhydryde are added and the mixture is stirred at 50°C for 24 h. After that, Dowex 50 x 4-400 was added until slightly acid pH. The Dowex is removed by filtration and washed with dichloromethane. The organic phase is washed several times with a 5% aqueous solution of sodium bicarbonate. The resulting aqueous phase is acidified with 1N chlorhydric acid and extracted with *tert*-butylmethylether. The organic phase is washed with a 10% aqueous solution of sodium chloride and concentrated to dryness to obtain the desired product. Yield: 0.92 g (64%). White solid, m.p: 148.9-151.2 °C.
- IR (cm⁻¹): 3445-2500, 1764, 1718, 1600, 1581, 1514.
- ¹H-NMR (CDCl₃), δ (ppm): 1.75-1.92 (m,2H); 2.23-2.37 (m, 1H); 2.45-2.60 (m, 1H); 2.69-2.84 (m, 1H); 3.01-3.12 (m, 1H); 3.70-3.77 (m, 1H); 4.12-4.16 (m, 1H); 4.36-4.44 (m, 1H); 4.63-4.71 (m, 1H); 6.21 (br s, 1H); 7.01 (dd, 2H); 7.09-7.25 (m, 5H); 7.31 (t, 2H); 7.41-7.50 (m, 2H); 7.61-7.69 (m, 2H).
- ¹³C-NMR (CDCl₃), δ (ppm): 34.6 (CH₂); 40.7 (CH); 44.5 (CH); 45.2 (CH₂); 48.1 (CH₂); 65.5 (CH₂); 115.6 (CH); 121.6 (CH); 125.4 (CH); 128.2 (CH); 128.6 (CH); 129.2 (CH); 130.0 (CH); 130.4 (C); 131.2 (CH); 137.9 (C); 150.9 (C); 154.0 (CO); 161.7 (C); 167.5 (CO); 171.2 (CO).
- E.M.-E.S.I+: 478 (M + H, 2%); 500 (M + Na, 100); 516 (M + K, 15)

### Example 6

### (±)-trans-3-[Carboxyethanoyloxymethyl]-4-(4'-fluorophenyl)-N-pfenyloxycarbonyl-piperidine. (IV, R³ = Ph, R⁴ = CH₂)

1 g of (±)-*trans*-4-(4'-Fluorophenyl)-3-hydroxymethyl-*N*-phenyloxycarbonylpiperidine is dissolved in 40 ml dichloromethane and 1 mL pyridine. 1.74 g of Meldrum's acid are added and the mixture is stirred at 50°C for 24 h. After that, Dowex 50 x 4-400 was added until slightly acid pH. The Dowex is removed by filtration and washed with dichloromethane. The organic phase is washed several times with a 5% aqueous solution of sodium bicarbonate. The resulting aqueous phase is acidified with 1N chlorhydric acid and extracted with *tert*-butylmethylether. The organic phase is washed with a 10% aqueous solution of sodium chloride and concentrated to dryness to obtain the desired product. Yield: 0.65 g (52%). Hygroscopic solid.
- IR (cm⁻¹): 3450-2700, 1742, 1715, 1605, 1510.
- ¹H-NMR (CDCl₃), δ (ppm): 1.73-1.96 (m,2H); 2.09-2.25 (m, 1H); 2.59 (td, 1H); 2.73-3.06 (m, 1H); 3.34 (s, 2H); 3.74 (dd, 1H); 3.96-4.05 (m, 1H); 4.38-4.59 (m, 2H); 7.02 (dd, 2H); 7.08-7.24 (m, 6H); 7.39 (t, 2H).
- ¹³C-NMR (CDCl₃), δ (ppm): 33.9 (CH₂); 40.6 (CH); 40.9 (CH₂); 44.1 (CH); 44.3 (CH₂); 47.5 (CH₂); 65.3 (CH₂); 115.5 (CH); 121.2 (CH); 125.6 (CH); 128.5 (CH); 129.0 (CH); 138.1 (C); 151.4 (C); 153.6 (CO); 161.2 (C); 166.0 (CO); 168.7 (CO).
- E.M.-E.S.I+: 416 (M + H, 7%); 438 (M + Na, 100); 454 (M + K, 30)

### Example 7

### Enzymatic resolution of the intermediate III. (R³= Ph, R⁴= (CH₂)₂).

0.1 g of (±)-*trans*-4-(4'-Fluorophenyl)-3-hydroxymethyl-*N*-phenyloxycarbonylpiperidine is dissolved in a suspension of 8 ml toluene, 0.06 g of succinic anhydryde and 0.125 g of lipase CAL-A. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*R*, 4*S*)-*trans*-4-(4'-Fluorophenyl)-3-hydroxymethyl-*N*-phenyloxycarbonylpiperidine (61% ee). The resulting aqueous phase is acidified with 1N chlorhydric acid and extracted with *tert*-butylmethylether. The organic phase is washed with a 10% aqueous solution of sodium chloride and concentrated to dryness to obtain (3*S*,4*R*)-3-[(3-Carboxypropanoyl)oxymethyl]-4-(4'-fluorophenyl)-N-phenyloxycarbonylpiperidine (93% ee; [α]_{D}¹⁸ = -3.08, c 0.64, MeOH). Conversion: 40%.

### Example 8

### Enzymatic resolution of the intermediate III. (R³= Ph, R⁴= (CH₂)₂).

0.1 g of (±)-*trans*-4-(4'-Fluorophenyl)-3-hydroxymethyl-*N*-phenyloxycarbonylpiperidine is dissolved in a suspension of 8 ml toluene, 0.06 g of succinic anhydryde and 0.125 g of lipase CAL-B. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (46% ee). The resulting aqueous phase is acidified with 1N chlorhydric acid and extracted with *tert*-butylmethylether. The organic phase is washed with a 10% aqueous solution of sodium chloride and concentrated to dryness to obtain (3*R*,4*S*)-3-[(3-Carboxypropanoyl)oxymethyl]-4-(4'-fluorophenyl)-*N*-phenyloxycarbonylpiperidine (92% ee; [α]_{D}¹⁸ = +3.18, c 0.75, MeOH). Conversion: 33%.

### Example 9

### Enzymatic resolution of the intermediate III. (R³= Ph, R⁴= (CH₂)₂).

0.1 g of (±)-*trans*-4-(4'-Fluorophenyl)-3-hydroxymethyl-*N*-phenyloxycarbonylpiperidine is dissolved in a suspension of 8 ml toluene, 0.06 g of succinic anhydryde and 0.110 g of lipase PS-C. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (3% ee). The resulting aqueous phase is acidified with 1 N chlorhydric acid and extracted with *tert*-butylmethylether. The organic phase is washed with a 10% aqueous solution of sodium chloride and concentrated to dryness to obtain (3*R*,4*S*)-3-[(3-Carboxypropanoyl)oxymethyl]-4-(4'-fluorophenyl)-*N*-phenyloxycarbonylpiperidine (40% ee; Conversion: 7%.

### Example 10

### Enzymatic resolution of the intermediate III. (R³= Ph, R⁴= (CH₂)₂).

0.1 g of (±)-*trans*-4-(4'-Fluorophenyl)-3-hydroxymethyl-*N*-phenyloxycarbonylpiperidine is dissolved in a suspension of 8 ml *tert*-butylmethylether, 0.06 g of succinic anhydryde and 0.125 g of lipase CAL-A. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*R*, 4*S*)-*trans*-4-(4'-Fluorophenyl)-3-hydroxymethyl-*N*-phenyloxycarbonylpiperidine (29% ee). The resulting aqueous phase is acidified with 1N chlorhydric acid and extracted with *tert*-butylmethylether. The organic phase is washed with a 10% aqueous solution of sodium chloride and concentrated to dryness to obtain (3*S*,4*R*)-3-[(3-Carboxypropanoyl)oxymethyl]-4-(4'-fluorophenyl)-*N*-phenyloxycarbonylpiperidine (91% ee). Conversion: 24%.

### Example 11

### Enzymatic resolution of the intermediate III. (R³= Ph, R⁴= (CH₂)₃).

0.1 g of (±)-*trans*-4-(4'-Fluorophenyl)-3-hydroxymethyl-*N*-phenyloxycarbonylpiperidine is dissolved in a suspension of 8 ml toluene, 0.07 g of glutaric anhydryde and 0.125 g of lipase CAL-A. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*R*, 4*S*)-*trans*-4-(4'-Fluorophenyl)-3-hydroxymethyl-*N*-phenyloxycarbonylpiperidine (35% ee). The resulting aqueous phase is acidified with 1N chlorhydric acid and extracted with *tert*-butylmethylether. The organic phase is washed with a 10% aqueous solution of sodium chloride and concentrated to dryness to obtain (3*S*,4*R*)-*trans*-3-[(4-Carboxybutanoyl)oxymethyl]-4-(4'-fluorophenyl)-*N*-phenyloxycarbonylpiperidine (77% ee; [α]_{D}¹⁸ = - 2.52, c 0.56, MeOH). Conversion: 32%.

### Example 12

### Enzymatic resolution of the intermediate III. (R³= Ph, R⁴= (CH₂)₃).

0.1 g of (±)-*trans*-4-(4'-Fluorophenyl)-3-hydroxymethyl-*N*-phenyloxycarbonylpiperidine is dissolved in a suspension of 8 ml toluene, 0.03 g of glutaric anhydryde and 0.125 g of lipase CAL-B. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (54% ee). The resulting aqueous phase is acidified with 1N chlorhydric acid and extracted with *tert*-butylmethylether. The organic phase is washed with a 10% aqueous solution of sodium chloride and concentrated to dryness to obtain (3*R*,4*S*)-trans-3-[(4-Carboxybutanoyl)oxymethyl]-4-(4'-fluorophenyl)-*N*-phenyloxycarbonylpiperidine (96% ee; [α]_{D}¹⁸ = +2.84, c 0.84, MeOH). Conversion: 36%.

### Example 13

### Enzymatic resolution of the intermediate III. (R³= Ph, R⁴= (CH₂)₃).

0.1 g of (±)-*trans*-4-(4'-Fluorophenyl)-3-hydroxymethyl-*N*-phenyloxycarbonylpiperidine is dissolved in a suspension of 8 ml *tert*-butylmethylether, 0.07 g of glutaric anhydryde and 0.125 g of lipase CAL-B. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (93% ee). The resulting aqueous phase is acidified with 1N chlorhydric acid and extracted with *tert*-butylmethylether. The organic phase is washed with a 10% aqueous solution of sodium chloride and concentrated to dryness to obtain (3*R*,4*S*)-*trans*-3-[(4-Carboxybutanoyl)oxymethyl]-4-(4'-fluorophenyl)-*N*-phenyloxycarbonylpiperidine (83% ee). Conversion: 53%.

### Example 14

### Enzymatic resolution of the intermediate III. (R³= Ph, R⁴= (CH₂)₃).

0.1 g of (±)-*trans*-4-(4'-Fluorophenyl)-3-hydroxymethyl-*N*-phenyloxycarbonylpiperidine is dissolved in a suspension of 8 ml toluene, 0.07 g of glutaric anhydryde and 0.125 g of lipase CAL-B. The mixture was shaken at 15°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (50% ee). The resulting aqueous phase is acidified with 1N chlorhydric acid and extracted with *tert*-butylmethylether. The organic phase is washed with a 10% aqueous solution of sodium chloride and concentrated to dryness to obtain (3*R*,4*S*)-*trans*-3-[(4-Carboxybutanoyl)oxymethyl]-4-(4'-fluorophenyl)-*N*-phenyloxycarbonylpiperidine (96% ee). Conversion: 34%.

### Example 15

Enzymatic resolution of the intermediate III. (R³= Ph, R⁴= (CH₂OCH₂).

0.1 g of (±)-*trans*-4-(4'-Fluorophenyl)-3-hydroxymethyl-*N*-phenyloxycarbonylpiperidine is dissolved in a suspension of 8 ml *tert*-butylmethylether, 0.07 g of diglycolic anhydryde and 0.125 g of lipase CAL-B. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (33% ee). The resulting aqueous phase is acidified with 1N chlorhydric acid and extracted with *tert*-butylmethylether. The organic phase is washed with a 10% aqueous solution of sodium chloride and concentrated to dryness to obtain (3*R*,4*S*)-*trans*-3-[(4-Carboxy-3-oxabutanoyl)oxymethyl]-4-(4'-fluorophenyl)-*N*-phenyloxycarbonylpiperidine (30% ee; [α]_{D}¹⁸ = +0.75, c 1.40, MeOH). Conversion: 52%.

### Ejemplo 16

### Enzymatic resolution of the intermediate III. (R³= Ph, R⁴= (CH₂OCH₂).

0.1 g of (±)-*trans*-4-(4'-Fluorophenyl)-3-hydroxymethyl-*N*-phenyloxycarbonylpiperidine is dissolved in a suspension of 8 ml *tert*-butylmethylether, 0.07 g of diglycolic anhydryde and 0.125 g of lipase CAL-A. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*R*, 4*S*)-*trans*-4-(4'-Fluorophenyl)-3-hydroxymethyl-*N*-phenyloxycarbonylpiperidine (3.0% ee). The resulting aqueous phase is acidified with 1N chlorhydric acid and extracted with *tert*-butylmethylether. The organic phase is washed with a 10% aqueous solution of sodium chloride and concentrated to dryness to obtain (3*S*,4*R*)-*trans*-3-[(4-Carboxy-3-oxabutanoyl)oxymethyl]-4-(4'-fluorophenyl)-*N*-phenyloxycarbonylpiperidine (43% ee; [α]_{D}¹⁸ = +0.93, c 1.12, MeOH). Conversion: 12%.

### Example 17

### Enzymatic resolution of the intermediate III. (R³= Ph, R⁴= (CH₂).

0.1 g of (±)-*trans*-4-(4'-Fluorophenyl)-3-hydroxymethyl-*N*-phenyloxycarbonylpiperidine is dissolved in a suspension of 8 ml diisopropyl ether, 0.09 g of Meldrum's acid and 0.125 g of lipase CAL-A. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*R*, 4*S*)-*trans*-4-(4'-Fluorophenyl)-3-hydroxymethyl-*N*-phenyloxycarbonylpiperidine (16% ee). The resulting aqueous phase is acidified with 1N chlorhydric acid and extracted with *tert*-butylmethylether. The organic phase is washed with a 10% aqueous solution of sodium chloride and concentrated to dryness to obtain (3*S*,4*R*)-3-[Carboxyethanoyloxymethyl]-4-(4'-fluorophenyl)-*N*-phenyloxycarbonylpiperidine (39% ee; [α]_{D}¹⁸ = - 1.12, c 1.30, MeOH). Conversion: 29%.

### Ejemplo 18

### Enzymatic resolution of the intermediate III. (R³= Ph, R⁴= (CH₂).

0.1 g of (±)-*trans*-4-(4'-Fluorophenyl)-3-hydroxymethyl-*N*-phenyloxycarbonylpiperidine is dissolved in a suspension of 8 ml *tert*-butylmethyl ether, 0.09 g of Meldrum's acid and 0.125 g of lipase CAL-B. The mixture was shaken at 30°C. When the desired conversion is reached, the enzyme is filtered and concentrated to dryness. The resulting oil is dissolved in dichloromethane and extracted several times with a 5% aqueous solution of sodium bicarbonate. The resulting organic phase is concentrated to dryness to obtain (3*S*, 4*R*)-*trans*-4-(4'-Fluorophenyl)-3-hydroxymethyl-*N*-phenyloxycarbonylpiperidine (31% ee). The resulting aqueous phase is acidified with 1N chlorhydric acid and extracted with *tert*-butylmethylether. The organic phase is washed with a 10% aqueous solution of sodium chloride and concentrated to dryness to obtain (3*R*,4*S*)-3-[Carboxyethanoyloxymethyl]-4-(4'-fluorophenyl)-*N*-phenyloxycarbonylpiperidine (34% ee; [α]_{D}¹⁸ = +0.98, c 0.95, MeOH). Conversion: 48%.

### Example 19

### (±)-trans-4-(4'-Fluorophenyl)-3-[(3-methoxycarbonylpropanoyl)oxymethyl]-N-phenyloxycarbonylpiperidine. (V, R³ = Ph, R⁴ = (CH₂)₂)

1.0 g of (±)-*trans*-3-[(3-carboxypropanoyl)oxymethyl]-4-(4'-fluorophenyl)-*N*-phenyloxycarbonylpiperidine are disolved in 50 mL of dry THF. 3.3 mL of dry MeOH and 3.3 mL of a 2.0 M solution of trimethylsilyldiazomethane in hexane are slowly added to the mixture. After 2 hours stirring at room temperature, the solvents are evaporated to dryness. The resulting oil is purified by flash chromatography on silica gel. Yield: 0.81 g (79%), oil.
- IR (cm⁻¹): 1734, 1714, 1605, 1510.
- ¹H-NMR (CDCl₃), δ (ppm): 1.78-1.89 (m, 2H); 2.09-2.22 (m, 1H); 2.52-2.66 (m, 5H); 2.81-3.11 (m, 2H); 3.69-3.78 (m, 4H); 3.93-3.97 (m, 1H); 4.41-4.56 (m, 2H); 7.03 (dd, 2H); 7.07-7.24 (m, 5H); 7.42 (t, 2H).
- ¹³C-NMR (CDCl₃), δ (ppm): 28.6 (CH₂); 28.8 (CH₂); 34.1 (CH₂); 40.8 (CH); 44.3 (CH); 44.7 (CH₂); 47.6 (CH₂); 51.8 (CH₃); 64.6 (CH₂); 115.6 (CH); 121.6 (CH); 125.2 (CH); 128.5 (CH); 129.2 (CH); 138.2 (C); 151.2 (C); 153.5 (CO); 161.6 (C); 171.8 (CO); 172.5 (CO).
- E.M.-E.S.I+: 444 (M + H, 18%); 466 (M + Na, 100); 482 (M + K, 53).

### Example 20

### (±)-trans-4-(4'-Fluorophenyl)-3-[(4-methoxycarbonylbutanoyl)oxymethyl]-N-phenyloxycarbonylpiperidine. (V, R³ = Ph, R⁴ = (CH₂)₃)

1.0 g of (±)-*trans*-3-[(4-carboxybutanoyl)oxymethyl]-4-(4'-fluorophenyl)-*N*-phenyloxycarbonylpiperidine are disolved in 50 mL of dry THF. 3.2 mL of dry MeOH and 3.2 mL of a 2.0 M solution of trimethylsilyldiazomethane in hexane are slowly added to the mixture. After 2 hours stirring at room temperature, the solvents are evaporated to dryness. The resulting oil is purified by flash chromatography on silica gel. Yield: 0.75 g (73%), oil.
- IR (cm⁻¹): 1742, 1717, 1600, 1508.
- ¹H-NMR (CDCl₃), δ (ppm): 1.82-1.99 (m, 4H); 2.13-2.19 (m, 1H); 2.35 (t, 4H); 2.51-2.60 (m, 1H); 2.74-3.10 (m, 2H); 3.66-3.78 (m, 4H); 3.90-3.97 (m, 1H); 4.33-4.55 (m, 2H); 7.03 (dd, 2H); 7.14-7.25 (m, 5H); 7.39 (t, 2H).
- ¹³C-NMR (CDCl₃), δ (ppm): 19.9 (CH₂); 32.8 (CH₂); 32.9 (CH₂); 33.4 (CH₂); 40.8 (CH); 44.4 (CH); 44.7 (CH₂); 47.9 (CH₂); 51.5 (CH₃); 64.4 (CH₂); 115.6 (CH); 121.6 (CH); 125.3 (CH); 128.6 (CH); 129.2 (CH); 138.2 (C); 151.2 (C); 153.5 (CO); 161.7 (C); 172.5 (CO); 173.9 (CO).
- E.M.-E.S.I+: 458 (M + H, 10%); 480 (M + Na, 100); 496 (M + K, 30).

### Ejemplo 21

### (±)-trans-4-(4'-Fluorophenyl)-3-[(4-methoxycarbonyl-3-oxabutanoyl)oxymethyl]-N-phenyloxycarbonylpiperidine. (V, R³ = Ph, R⁴ = (CH₂OCH₂)

1.0 g of (±)-*trans*-4-(4'-Fluorophenyl)-3-[(4-methoxycarbonyl-3-oxabutanoyl)oxymethyl]-*N*-phenyloxycarbonylpiperidine are disolved in 50 mL of dry THF. 3.2 mL of dry MeOH and 3.2 mL of a 2.0 M solution of trimethylsilyldiazomethane in hexane are slowly added to the mixture. After 2 hours stirring at room temperature, the solvents are evaporated to dryness. The resulting oil is purified by flash chromatography on silica gel. Yield: 0.83 g (81 %), hygroscopic solid.
- IR (cm⁻¹): 1746, 1714, 1605, 1512.
- ¹H-NMR (CDCl₃), δ (ppm): 1.70-1.89 (m, 2H); 2.09-2.21 (m, 1H); 2.54 (td, 1H); 2.71-3.03 (m, 2H); 3.72-3.82 (m, 4H); 3.95-4.02 (m, 1H); 4.15-4.21 (m, 4H); 4.37-4.51 (m, 2H); 7.01 (dd, 2H); 7.13-7.23 (m, 5H); 7.37 (t, 2H).
- ¹³C-NMR (CDCl₃), δ (ppm): 33.7 (CH₂); 40.7 (CH); 44.3 (CH); 44.7 (CH₂); 47.1 (CH₂); 51.8 (CH₃); 64.84 (CH₂); 67.7 (CH₂); 67.9 (CH₂); 115.6 (CH); 121.6 (CH); 125.2 (CH); 128.5 (CH); 129.2 (CH); 138.1 (C); 151.2 (C); 153.5 (CO); 161.6 (C); 169.3 (CO); 169.8 (CO).
- E.M.-E.S.I+: 460 (M + H, 2%); 482 (M + Na, 100); 498 (M + K, 10).

### Ejemplo 22

### (±)-trans-4-(4'-Fluorophenyl)-3-[(3-methoxycarbonylprop-2-enoyl)oxymethyl]-N-phenyloxycarbonylpiperidine. (V, R³ = Ph, R⁴ = (CH=CH)

1.0 g of (±)-*trans*-3-[(3-carboxylprop-2-enoyl)oxymethyl]-4-(4'-Fluorophenyl)-*N*-phenyloxycarbonylpiperidine are disolved in 50 mL of dry THF. 3.3 mL of dry MeOH and 3.3 mL of a 2.0 M solution of trimethylsilyldiazomethane in hexane are slowly added to the mixture. After 2 hours stirring at room temperature, the solvents are evaporated to dryness. The resulting oil is purified by flash chromatography on silica gel. Yield: 0.73 g (71%), oil.
- IR (cm⁻¹): 1739, 1724, 1650, 1598, 1502.
- ¹H-RMN (CDCl₃), δ (ppm): 1.83-2.02 (m, 2H); 2.14-2.25 (m, 1H); 2.60 (td, 1H); 2.78-3.12 (m, 2H); 3.76 (s, 3H); 3.86 (dd, 1H); 4.01-4.08 (m, 1H); 4.40-4.59 (m, 2H); 6.22 (m, 2H); 7.04 (dd, 2H); 7.13-7.26 (m, 5H); 7.39 (t, 2H).
- ¹³C-RMN (CDCl₃), δ (ppm): 33.5 (CH₂); 40.4 (CH); 44.1 (CH); 44.6 (CH₂); 46.91 (CH₂); 51.7 (CH₃); 64.8 (CH₂); 115.2 (CH); 121.2 (CH); 124.8 (CH); 128.1 (CH); 128.2 (CH); 128.6 (CH); 129.2 (CH); 137.7 (C); 150.8 (C); 153.1 (CO); 161.2 (C); 164.5 (CO); 164.9 (CO).
- E.M.-E.S.I+: 442 (M + H, 5%); 464 (M + Na, 100); 480 (M + K, 23).

### Example 23

### (±)-trans-4-(4'-Fluorophenyl)-3-[(2-methoxycarbonylbenzoyl)oxymethyl]-N-phenyloxycarbonylpiperidine. (V, R³ = Ph, R⁴ = o-phenylene)

1.0 g of (±)-*trans*--3-[(2-carboxybenzoyl)oxymethyl]-4-(4'-Fluorophenyl)-*N*-phenyloxycarbonylpiperidine are disolved in 50 mL of dry THF. 2.9 mL of dry MeOH and 2.9 mL of a 2.0 M solution of trimethylsilyldiazomethane in hexane are slowly added to the mixture. After 2 hours stirring at room temperature, the solvents are evaporated to dryness. The resulting oil is purified by flash chromatography on silica gel. Yield: 0.64 g (62%). White solid, m.p: 138.9-141.0 °C.
- IR (cm⁻¹): 1764, 1718, 1600, 1584, 1514.
- ¹H-RMN (CDCl₃), δ (ppm): 1.84-1.99 (m, 2H); 2.23-2.31 (m, 1H); 2.66 (td, 1H); 2.81-3.12 (m, 2H); 3.86 (s, 3H); 3.97 (dd, 1H); 4.08-4.15 (m, 1H); 4.39-4.48 (m, 1H); 4.56-4.67 (m, 1H), 7.05 (dd, 2H); 7.10-7.25 (m, 5H); 7.39 (t, 2H), 7.51-7.56 (m, 2H), 7.62-7.6 (m, 1H), 7.72-7.77 (m, 1H).
- ¹³C-RMN (CDCl₃), δ (ppm): 34.2 (CH₂); 41.1 (CH); 44.3 (CH); 44.9 (CH₂); 47.4 (CH₂); 52.6 (CH₃); 65.6 (CH₂); 115.7 (CH); 121.6 (CH); 125.3 (CH); 128.4 (CH); 128.6 (CH); 128.9 (CH); 129.2 (CH); 131.1 (C); 131.2 (CH); 138.2 (C); 151.3 (C); 153.6 (CO); 161.7 (C); 167.4 (CO); 167.7 (CO).
- E.M.-E.S.I+: 492 (M + H, 4%); 514 (M + Na, 100); 530 (M + K, 23).

### Example 24

### (±)-trans-4-(4'-Fluorophenyl)-3-[methoxycarbonylethanoyloxymethyl]-N-phenyloxycarbonylpiperidine. (V, R³ = Ph, R⁴ = CH₂)

1.0 g of (±)-*trans*-3-[carboxyethanoyloxymethyl]-4-(4'-Fluorophenyl)-*N*-phenyloxycarbonylpiperidine are disolved in 50 mL of dry THF. 3.4 mL of dry MeOH and 3.4 mL of a 2.0 M solution of trimethylsilyldiazomethane in hexane are slowly added to the mixture. After 2 hours stirring at room temperature, the solvents are evaporated to dryness. The resulting oil is purified by flash chromatography on silica gel. Yield: 0.78 g (76%), oil.
- IR (cm⁻¹): 1733, 1717, 1603, 1509.
- ¹H-RMN (CDCl₃), δ (ppm): 1.79-1.92 (m, 2H); 2.15-2.23 (m, 1H); 2.59 (td, 1H); 2.73-3.07 (m, 2H); 3.37 (s, 2H); 3.78-3.84 (m, 4H); 3.94-3.99 (m, 1H); 4.45-4.62 (m, 2H); 7.05 (dd, 2H); 7.12-7.24 (m, 5H); 7.40 (t, 2H).
- ¹³C-RMN (CDCl₃), δ (ppm): 33.9 (CH₂); 40.6 (CH); 41.0 (CH₂); 44.2 (CH); 44.7 (CH₂); 47.2 (CH₂); 52.4 (CH₃); 65.3 (CH₂); 115.6 (CH); 121.6 (CH); 125.2 (CH); 128.5 (CH); 129.2 (CH); 138.1 (C); 151.2 (C); 153.5 (CO); 161.6 (C); 166.0 (CO); 166.6 (CO).
- E.M.-E.S.I+: 430 (M + H, 10%); 452 (M + Na, 100); 468 (M + K, 72).

### Example 25

### (3S,4R)-trans-4-(4'-Fluorophenyl)-3-hydroxymethyl-N-phenyloxycarbonylpiperidine

0.1 g of (3*S*,4*R*)-*trans*-4-(4'-Fluorophenyl)-3-[(3-methoxycarbonylpropanoyl)oxymethyl]-*N*-phenyloxycarbonylpiperidine are dissolved in 5 mL of a 2.0 M aqueous solution of NaOH. The mixture is stirred for 2 hours at room temperature and then, extracted with toluene. The organic phase is washed with a 10% aqueous solution of sodium chloride and concentrated to dryness. Yield: 0.06 g (76%).

## Claims

1. A *trans*-*N*-alcoxycarbonyl-4-aryl-3-acyloxymethylpiperidine of formula IV, with configuration (3*S*, 4*R*) or (3*R*, 4*S*) or a mixture of both, where:
• R³ is: alkyl, alkenyl, aralkyl or aryl.
• R⁴ is: alkyl, alkyloxyalkyl, alkenyl or aryl.
• Ar is: phenyl substituted by one or several halogen atoms.

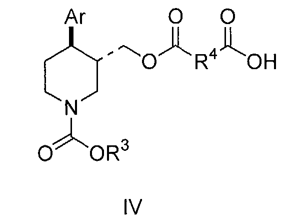

2. A *trans*-*N*-alcoxycarbonyl-4-aryl-3-acyloxymethylpiperidine of formula V, with configuration (3*S*, 4*R*) or (3*R*, 4*S*) or a mixture of both, where:
• R³ is: alkyl, alkenyl, aralkyl or aryl.
• R⁴ is: alkyl, alkyloxyalkyl, alkenyl or aryl.
• Ar is: phenyl substituted by one or several halogen atoms.

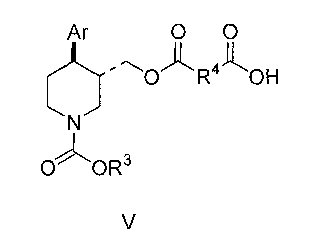

3. A process to prepare a 4-aryl-3-acyloxymethylpiperidine according to claim 1 and a 4-aryl-3-hydroxymethylpiperidine of formula III, both optically active, **characterised by**:
• stereospecifically acylating a mixture of the isomers (+) and (-) of a compound of formula III using an acylating agent that can be a cyclic anhydryde or the Meldrum's acid and an enzyme,
• stopping the reaction at a determined conversion, less than 100%, and
• separating the compound of formula (3*S*, 4*R*)-IV obtained from the remaining compound (3*R*, 4*S*)-III. 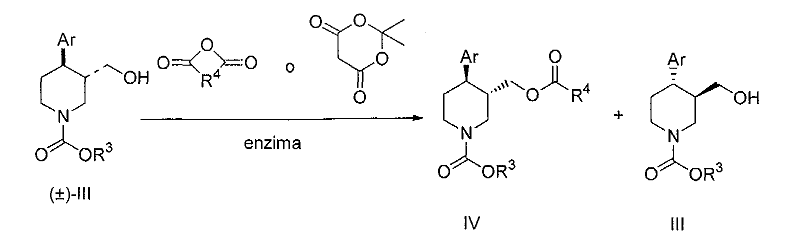
where:
• R³ is: alkyl, alkenyl, aralkyl or aryl.
• R⁴ is: alkyl, alkyloxyalkyl, alkenyl or aryl.
• Ar is: phenyl substituted by one or several halogen atoms.

4. A process to prepare a 4-aryl-3-acyloxymethylpiperidine according to claim 1 and a 4-aryl-3-hydroxymethylpiperidine of formula III, both optically active, **characterised by**:
• stereospecifically acylating a mixture of the isomers (+) and (-) of a compound of formula III using an acylating agent that can be a cyclic anhydryde or the Meldrum's acid and an enzyme,
• stopping the reaction at a determined conversion, less than 100%, and
• separating the compound of formula (3*R*, 4*S*)-IV obtained from the remaining compound (3*S*, 4*R*)-III. 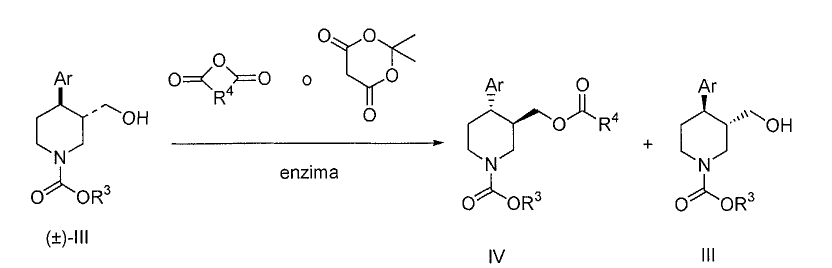
where:
• R³ is: alkyl, alkenyl, aralkyl or aryl.
• R⁴ is: alkyl, alkyloxyalkyl, alkenyl or aryl.
• Ar is: phenyl substituted by one or several halogen atoms.

5. A process according to claims 3 and 4, **characterised in that** the final enantiomeric excess of compound IV is greater than 60%.

6. A process according to claims 3 and 4, **characterised in that** the final enantiomeric excess of compound IV is greater than 95%.

7. A process according to claims 3 and 4, **characterised in that** the final enantiomeric excess of compound III is greater than 60%.

8. A process according to claims 3 and 4, **characterised in that** the final enantiomeric excess of compound III is greater than 95%.

9. A process according to claims 3 and 4, **characterised in that** the variable part of the formula R³ is methyl, allyl, benzyl, *tert*-butyl or phenyl.

10. A process according to claims 3 and 4, **characterised in that** the variable part of the formula R⁴ is methylene ethylene, trimethylene, oxybismethylene, vynilene or o-phenylene.

11. A process according to claims 3 and 4, **characterised in that** the variable part of the formula Ar is 4-fluorophenyl.

12. A process according to claims 3 and 4, **characterised in that** the enzyme is a hydrolase.

13. A process according to claims 3 and 4, **characterised in that** the enzyme is a lipase.

14. A process according to claims 3 and 4, **characterised in that** the lipase comes from a micro-organism of the generes *Candida, Rhizomucor, Pseudomonas* or *Aspergillus.*

15. A process according to claims 3 and 4, **characterised in that** the lipase is CAL-A (lipase A of *Candida antarctica*), CAL-B (lipase B of *Candida antarctica*), PS-C (lipase of *Pseudomonas cepacia*).

16. A process according to claims 3 and 4, **characterised in that** the enzyme is partially or totally purified.

17. A process according to claims 3 and 4, **characterised in that** the enzyme is immobilised.

18. A process according to claims 3 and 4, **characterised in that** the reaction is performed in an organic solvent or in a mixture of organic solvents.

19. A process according to claims 3 and 4, **characterised in that** the reaction is performed in any solvent from toluene, diisopropylether or *tert*-butylmethylether.

20. A process to obtain a compound of formula (3*S*, 4*R*)-III, **characterised by** the deacylation (by hydrolysis, alcoholysis or ester aminolysis) of a compound of formula (3*S*, 4*R*)-IV obtained according to claim 3.

21. A process for the preparation of paroxetine, **characterised in that** it is obtained from a (3*S*, 4*R*)-III compound, in turn obtained according to either of the claims 4 or 20.

22. A process to obtain a compound of formula (3*S*, 4*R*)-V, **characterised by** the esterification of a compound of formula (3*S*, 4*R*)-IV obtained according to claim 3.

23. A process for the preparation of paroxetine, **characterised in that** it is obtained from a (3*S*, 4*R*)-IV compound, in turn obtained according to claim 3.

24. A process for the preparation of paroxetine, **characterised in that** it is obtained from a (3*S*, 4*R*)-V compound, in turn obtained according to claim 22.
